(19) Europäisches Patentamt
European Patent Office
Office européen des brevets

(11) **EP 4 040 444 A1**

(12) **EUROPEAN PATENT APPLICATION**

(43) Date of publication:
**10.08.2022 Bulletin 2022/32**

(21) Application number: **21179547.1**

(22) Date of filing: **15.06.2021**

(51) International Patent Classification (IPC):
**G16H 20/70** (2018.01)     **A61B 5/16** (2006.01)
**G16H 50/50** (2018.01)     **G16H 10/60** (2018.01)
**G06F 3/01** (2006.01)     **G06T 19/00** (2011.01)

(52) Cooperative Patent Classification (CPC):
**G16H 20/70; A61B 5/165; A61B 5/4836;**
A61B 5/01; A61B 5/14542; A61B 5/318;
A61B 5/389; G16H 10/60; G16H 50/50

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.02.2021 IT 202100002393**

(71) Applicant: **Virtual Factory S.r.l.**
**84091 Battipaglia (SA) (IT)**

(72) Inventors:
• **LONGO, Donato**
 **84091 Battipaglia (SA) (IT)**
• **MAINARDI, Luigi**
 **84091 Battipaglia (SA) (IT)**
• **CATALDO, Emilio**
 **84091 Battipaglia (SA) (IT)**
• **MORRETTA, Patrizia**
 **84091 Battipaglia (SA) (IT)**

(74) Representative: **Conversano, Gabriele**
**Laforgia, Bruni & Partners srl**
**Via Michele Garruba, 3**
**70122 Bari (IT)**

(54) **SYSTEM OF TREATMENT OF A PHOBIC PATHOLOGY BY MEANS OF A VIRTUAL ENVIRONMENT**

(57)     Device for phobia treatment, comprising:
- at least a virtual reality visor;
- at least a sensor configured to detect a patient physiological parameter;
- control means, configured to manage the virtual reality environment produced by said visor and to acquire in inlet data detected by said at least one sensor configured to detect a patient physiological parameter,

characterized in that
said control means are configured to introduce in said virtual reality environment at least a stimulation configured to activate a phobic reaction, to acquire in inlet by said at least sensor configured to detect a patient physiological parameter and data detected and to vary the intensity of said stimulation as a function of the data detected by said sensor.

EP 4 040 444 A1

## Description

The present Patent application relates to a system of control, monitoring and treatment of phobias.

In particular, the present Patent application relates to a method for phobia treatment by means of a virtual environment where stimulations of predetermined kind and adjustable intensity are provided as a function of a physiological feedback detected on the user.

State of the art

At the state of the art, there are known many methods for phobias treatment by means of virtual or augmented reality environments.

A first example is described in document JPH11155955, which describes a virtual reality device reproducing an image or sound and introducing the object of the phobia to the patient in a virtual environment by means of a stereo headset.

Other embodiments are described in documents CN109523638, CN111028919A.

Anyway, to the best of the present inventors' knowledge there is no device configured to communicate to the patient suffering from a phobia, who is subjected to an immersive environment, a stimulation able to activate a phobic reaction, of parametrized intensity as a function of a coefficient of intensity, whose extent is defined as a function of feedback values obtained by sensors detecting patient physiological parameters.

It is to be specified that the definition immersive environment is referred to environments able to create augmented reality experiences (enrichment of the human sensorial perception by means of information, usually enjoyable by means of different aids, which would not be perceptible by means of the five senses), mixed reality experiences (referred to situations and sceneries where real and virtual objects are combined creating experiences where both real and virtual visual representations of the space are perceived at the same time) and virtual reality experiences (techniques allowing to live sensorial experiences of real or fictitious places or objects by means of simulations enjoyable via visors or Head Mounted Display).

Description of the invention

The device for phobia treatment according to the invention comprises:

- at least a virtual reality visor;
- at least a sensor configured to detect at least a patient physiological parameter;
- control means, configured to manage the virtual reality environment produced by said visor and to acquire in inlet data detected by said at least one sensor configured to detect a patient physiological parameter.

The device comprises further a user interface configured to allow a user other than the one subjected to treatment by means of the visor, to view the data detected by said at least one sensor configured to detect physiological parameters, and/o other parameters calculated starting from said physiological parameters, and to allow said user other than the one subjected to treatment, to interact with said control means in order to determine the features of the immersive environment the person under treatment is subjected to, and/or the intensity of said stimulation. Said user interface comprises preferably a display and respective interaction means of touchscreen or traditional kind, according to any one of the various kinds known at the state of the art.

The device is characterized in that said control means are configured to introduce in said virtual reality environment at least a stimulation explicitly configured to activate a phobic reaction, to acquire in inlet the data detected by said at least one sensor configured to detect a patient physiological parameter and to vary the intensity of said stimulation as a function of the data detected by said sensor.

Said device comprises preferably a plurality of sensors configured to acquire patient physiological parameters.

In a preferred embodiment, said plurality of sensors comprises one or more of the following sensors: a thermometer for measuring body temperature, a sensor configured to measure the patient muscle activity, an electrocardiograph, a sensor for measuring oxygen saturation.

The device according to the invention is configured to acquire physiological parameter values detected by said sensors and to calculate a coefficient of physiological reaction, proportional to the reaction generated in the patient (it is practically a parameter indicating the intensity of the phobic reaction).

Moreover, the device is configured to compare said coefficient of physiological reaction with a statistical distribution of parameters indicating the intensity of phobic reaction, detected in a plurality of persons subjected to stimulations able to cause phobic reactions. The immersive environment created by means of said virtual reality visor is preferably an environment similar to environments commonly lived by the patient. As a function of the phobia to be treated, the environment can be an open square, a closed room, a public building or any other kind of environment where the phobia to be treated manifests itself commonly.

Similarly, the stimulation able to activate the phobic reaction will be variable as a function of the phobia to be treated: it can be a spider in case of arachnophobia, many people together in case of a phobia of social kind, an open space in case of agoraphobia, a small and closed room in case of claustrophobia. It is clear that this is a not limiting example of a list of the kinds of phobias that can be treated by means of the device and method of the present invention.

A common feature to the various kinds of treatment that can be carried out by the device according to the invention, regardless of the kind of phobia treated, is that said stimulation is described by means of at least a parameter, so that the intensity of the reaction generated is proportional to a coefficient of control.

Preferably, in order to make the experience more real, said stimulation is parametrized as a function of a plurality of parameters so that the intensity of the reaction generated is always proportional to a unique coefficient of control.

It is clear in this case that the kind of relation created is not biunivocal, and more combinations of parameters allowing to obtain the same intensity of the generated reaction can correspond to each value of the coefficient of control.

In a preferred embodiment, the value of coefficient of control is a linear combination of a plurality of parameters describing the stimulation, each one multiplied for an own weight, as it is schematically shown in the following relation where the values "Pn" are the values of the various descriptive parameters, the coefficients "a" to "n" are weights of each parameter and C is referred to the coefficient of control

$$C = a*P1 + b*P2 + ... + n*Pn$$

As a way of example, if the phobia to be treated is arachnophobia, the parameters can be: the number of spiders in the immersive environment (P1), the kind (P2) and dimension (P3) of each spider, how close (P4) the spider is from the patient.

Yet, as a way of not limiting example, if the phobia to be treated is the fear of closed places, the parameters can be the three space dimensions, the number and dimensions of exits provided, the lighting level.

So, the stimulations can be parametrized so that a single great spider at a middle distance; a single small spider but closer to the patient; a plurality of spiders very far from the patient can correspond alternately to the same value of the coefficient of control.

The utility of this multi-parameter description will be clear from the following explanation.

Preferably, but not limitingly, said coefficient of control in between 0 and 1.

After describing the device, it is now possible to describe the method according to the invention.

In fact, the device is configured to carry out the method for phobia treatment described in the following:

    100) exposing the patient to an immersive environment by means of said virtual reality visor;
    200) detecting the physiological parameters of said patient and calculating at least a parameter indicating the intensity of phobic reaction;
    300) introducing in said immersive environment a stimulation able to activate the phobic reaction object of treatment, the intensity of said stimulation being proportional to a coefficient of control between 0 and 1, and the value of said coefficient of control being equal to 0.1 at first;
    400) detecting the patient physiological parameters again after a first interval of treatment of predetermined duration and calculating said at least one parameter indicating the intensity of phobic reaction again;
    500) comparing the parameter calculated at point 400) with a statistical distribution of parameters indicating the intensity of phobic reaction, detected in a plurality of persons subjected to stimulations able to generate phobic reactions;
    600) in case the parameter indicating the intensity of phobic reaction detected is lower than a predetermined threshold level, increasing said coefficient of control by a predetermined quantity and going back to step 400).
    Said predetermined threshold level is reached preferably when the intensity of phobic reaction is lower than 10% compared to the relative intensity at 80th percentile of said statistical distribution. Preferably, moreover, this predetermined quantity increasing said coefficient of control is equal to 0.1.
    700) in case the parameter indicating the intensity of phobic reaction detected is higher or equal to said predetermined threshold level, keeping the value of said coefficient of control constant for a predetermined observation time;
    800) if at the end of said predetermined observation time, the parameter indicating the intensity of phobic reaction has not gone under said threshold level, reducing said stimulation introduced at step 300);
    900) if at the end of said predetermined observation time, the parameter indicating the intensity of phobic reaction has gone under said threshold level, increasing said coefficient of control by a predetermined quantity and going back to step 400).

The device is also configured to allow, during the whole treatment, an outer operator to monitor in real time the data acquired by said sensors, and to allow said outer operator to intervene manually on the intensity of said stimulation.

In this way, it is created an environment in which the patient is exposed artificially to the object of his own phobia in a relaxed state, and the desensitization with respect to the stimulation improves thanks to the prolonged exposition. So, it is obtained a safe, comfortable and efficient treatment of phobias, which can be administered to a phobic patient.

It is to be specified that the device is configured to determine again the configuration of the stimulation at each variation of the coefficient of control, by choosing a combination of parameters defining the stimulation among the various combinations of parameters corresponding to the value of coefficient of control used.

In this way, the patient is subjected to a plurality of different situations producing the same stimulation, thus desensitizing his phobia with respect to a wider range of possible situations.

According to another embodiment, said device comprises remote communication means configured to allow an operator not physically close to the patient to monitor said physiological parameters during treatment and to control the intensity and kind of said stimulation introduced in the immersive environment.

An example of calculation of the coefficient of physiological reaction follows.

In a preliminary step of treatment, a phobic stimulation of known intensity is shown to a patient, in the just described ways.

The patient physiological parameters (pulse, saturation, muscle activation) are measured both before exposition to the phobic stimulation (to determine the basal level of the various parameters) and during the exposition to the phobic stimulation.

It is individuated the physiological parameter that after exposition to phobic stimulation is subjected to the greatest percentage variation, thus individuating it as useful parameter for calculating the coefficient of physiological reaction.

For example, a patient can have a heart rate at rest of 80 bpm before exposition to phobic stimulation, and a heart rate of 120 bpm after exposition to phobic stimulation whose coefficient of control is 1.

The coefficient of physiological reaction is so 40 bpm for each unit of phobic stimulation.

In the test execution step, proceeding by increases.

The heart rates are obtained preferably as average measures on a cluster of measurement lasting at least 30 seconds, so that the effects of heart rate physiological variations are limited.

So, the normalized coefficient of physiological reaction is 4 bpm for each increase by 0.1 of the value of the intensity of the phobic stimulation. This will be considered as the predetermined threshold value of the step 700) of the described method.

**Claims**

1. Device for phobia treatment, comprising:

   - at least a virtual reality visor, configured to produce a virtual reality environment;
   - at least a sensor configured to detect at least a patient physiological parameter;
   - control means, configured to manage the virtual reality environment produced by said visor and to acquire in inlet data detected by said at least one sensor configured to detect a patient physiological parameter,

   **characterized in that**
   said control means are configured to introduce in said virtual reality environment at least a stimulation configured to activate a phobic reaction, to acquire in inlet by said at least sensor configured to detect a patient physiological parameter the respective data and to vary the intensity of said stimulation as a function of the data detected by said sensor.

2. Device according to claim 1, **characterized in that** it comprises further a user interface configured to allow a user other than the one subjected to treatment to view the data detected by said at least one sensor configured to detect physiological parameters, and/o other parameters calculated starting from said physiological parameters, and to allow said user other than the one subjected to treatment to interact with said control means in order to determine the features of the immersive environment the person under treatment is subjected to, and/or the intensity of said stimulation.

3. Device according to claim 1 or 2, **characterized in that** it comprises one or more of the following sensors: a thermometer for measuring body temperature, a sensor configured to measure the patient muscle activity, an electrocardiograph, a sensor for measuring oxygen saturation.

4. Device according to any one of the preceding claims, **characterized in that** it is configured to acquire physiological parameter values detected by said sensors and to calculate a coefficient of physiological reaction, proportional to the reaction generated in the patient, and to compare said coefficient of physiological reaction with a statistical distribution of parameters indicating the intensity of phobic reaction, detected in a plurality of persons subjected to stimulations able to cause phobic reactions.

5. Device according to any one of the preceding claims, **characterized in that** said stimulation is parametrized as a function of a plurality of parameters so that the intensity of the reaction generated is always proportional to a unique coefficient of control.

6. Device according to any one of the preceding claims, **characterized in that** the value of coefficient of control is a linear combination of a plurality of parameters describing the stimulation, each one multiplied for an own weight.

7. Device according to any one of the preceding claims, **characterized in that** it is configured to carry out the method for phobia treatment described in the following:

   100) exposing the patient to an immersive environment by means of said virtual reality visor;

200) detecting the physiological parameters of said patient and calculating at least a parameter indicating the intensity of phobic reaction;

300) introducing in said immersive environment a stimulation able to activate the phobic reaction object of treatment, the intensity of said stimulation being proportional to a coefficient of control between 0 and 1, and the value of said coefficient of control being equal to 0.1 at first;

400) detecting the patient physiological parameters again after a first interval of treatment of predetermined duration and calculating said at least one parameter indicating the intensity of phobic reaction again;

500) comparing the parameter calculated at point 400) with a statistical distribution of parameters indicating the intensity of phobic reaction, detected in a plurality of persons subjected to stimulations able to generate phobic reactions;

600) in case the parameter indicating the intensity of phobic reaction detected is lower than a predetermined threshold level, increasing said coefficient of control by a predetermined quantity and going back to step 400);

700) in case the parameter indicating the intensity of phobic reaction detected is higher or equal to said predetermined threshold level, keeping the value of said coefficient of control constant for a predetermined observation time;

800) if at the end of said predetermined observation time, the parameter indicating the intensity of phobic reaction has not gone under said threshold level, reducing said stimulation introduced at step 300);

900) if at the end of said predetermined observation time, the parameter indicating the intensity of phobic reaction has gone under said threshold level, increasing said coefficient of control by a predetermined quantity and going back to step 400).

8. Device according to any one of the preceding claims, comprising further remote communication means configured to allow an operator not physically close to the patient to monitor said physiological parameters during treatment and to control the intensity and kind of said stimulation introduced in the immersive environment.

9. Device according to any one of the preceding claims, comprising further a user interface configured to allow a user other than the one subjected to treatment by means of the visor, to view the data detected by said at least one sensor configured to detect physiological parameters, and/o parameters calculated starting from said data, and to interact with said control means in order to determine the features of the immersive environment the person under treatment is subjected to.

Europäisches
Patentamt

European
Patent Office

Office européen
des brevets

**EUROPEAN SEARCH REPORT**

Application Number

EP 21 17 9547

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 807 114 A (HODGES LARRY F [US] ET AL) 15 September 1998 (1998-09-15) * the whole document, in particular column 1, line 1 - column 12- line 60, and figures 1-15 * | 1-9 | INV. G16H20/70 A61B5/16 |
| Y | US 6 425 764 B1 (LAMSON RALPH J [US]) 30 July 2002 (2002-07-30) * figures 1-9 * * column 1, line 20 - line 27 * * column 12, line 28 - column 19, line 31 * | 1-9 | ADD. G16H50/50 G16H10/60 G06F3/01 G06T19/00 |
| Y | EMMELKAMP PAUL M. G. ET AL: "Virtual Reality Treatment in Acrophobia: A Comparison with Exposure in Vivo", CYBERPSYCHOLOGY & BEHAVIOR, vol. 4, no. 3, 30 June 2001 (2001-06-30), pages 335-339, XP055852014, US ISSN: 1094-9313, DOI: 10.1089/109493101300210222 * abstract * * section "2. Method"; page 510 - page 512 * * section "5. Discussion"; page 514 - page 515 * | 1-9 | |

TECHNICAL FIELDS SEARCHED (IPC)

G16H
A61B
G06T
G06F

The present search report has been drawn up for all claims

| Place of search | Date of completion of the search | Examiner |
|---|---|---|
| Munich | 1 December 2021 | Sasa Bastinos, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 17 9547

This annex lists the patent family members relating to the patent documents cited in the above-mentioned European search report.
The members are as contained in the European Patent Office EDP file on
The European Patent Office is in no way liable for these particulars which are merely given for the purpose of information.

01-12-2021

| Patent document cited in search report | | Publication date | Patent family member(s) | | Publication date |
|---|---|---|---|---|---|
| US 5807114 | A | 15-09-1998 | AU | 2423697 A | 29-10-1997 |
| | | | US | 5807114 A | 15-09-1998 |
| | | | WO | 9737738 A1 | 16-10-1997 |
| US 6425764 | B1 | 30-07-2002 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H11155955 B
- CN 109523638

- CN 111028919 A